Europäisches Patentamt

European Patent Office

Office européen des brevets

(11) Publication number: **0 407 711 A1**

## EUROPEAN PATENT APPLICATION

(21) Application number: 90109367.4

(22) Date of filing: 17.05.90

(51) Int. Cl.5: **C07C 21/18**, C07C 17/34, B01J 27/128

| | |
|---|---|
| The title of the invention has been amended (Guidelines for Examination in the EPO, A-III, 7.3). | (71) Applicant: **ATOCHEM NORTH AMERICA, INC. Three Parkway Philadelphia, Pennsylvania 19102(US)** |
| (30) Priority: 28.06.89 US 373124 | (72) Inventor: **Elsheikh, Maher Yousef 784 North Wayne Avenue Wayne, Pennsylvania 19087(US)** |
| (43) Date of publication of application: **16.01.91 Bulletin 91/03** | |
| (84) Designated Contracting States: **BE DE FR GB IT NL** | (74) Representative: **Kraus, Walter, Dr. et al Patentanwälte Kraus, Weisert & Partner Thomas-Wimmer-Ring 15 15 D-8000 München 22(DE)** |

(54) Catalytic process for the dehydrohalogenation of 1-chloro-1,1-difluoroethane.

(57) 1-Chloro-1- fluoroethylene/vinylidene fluoride mixtures are prepared from the gas phase disproportionation of 1-chloro-1,1-difluoroethane in the presence of a $NiF_2$-containing catalyst.

EP 0 407 711 A1

# GAS PHASE CATALYTIC DISPROPORTIONATION OF 1-CHLORO-1,1-DIFLUOROETHANE

## Field of the Invention

The invention relates to the gas phase production of vinylidene fluoride and 1-chloro-1-fluoroethylene, in particular the production of vinylidene fluoride and 1-chloro-1-fluoroethylene by a gas phase catalytic disproportionation process.

## Background of the Invention

The gas phase catalytic dehydrohalogenation of 1-chloro-1,1-difluoroethane has been reported to proceed with elimination of HCl, with very little dehydrofluorination. Walker, et al., J. Org. Chem. 30, 3284-3285 (1965). The process was carried out between 300-400°C utilizing silver metal, NiO, $Fe_2O_3$ or ZnO. When $Al_2O_3$ or $SiO_2$ was used as the catalyst, the main product was 1-chloro-1-fluoroethylene, which resulted from the elimination of HF. The minor product, vinylidene fluoride, resulted from the elimination of HCl. A mixture of silica and alumina catalyst (12.4 wt.% $Al_2O_3$ and 87.3 wt.% $SiO_2$) resulted in both dehydrochlorination and dehydrofluorination, with the product distribution depending upon the temperature. Analysis of the oxide catalysts indicates that 5-10% of the catalyst reacted with HF or HCl. Such catalyst consumption reduces both catalyst lifetime and activity.

Müller, et al., **Chem. Ing. Tech.** 56 (8), 626-628 (1984) discloses dehydrochlorination of 1-chloro-1,1-difluoroethane in the presence of anhydrous $NiCl_2$. The process was very selective at 400°C. A small percentage of oxygen (1.5%), based upon the volume of 1-chloro-1,1-difluoroethane feed, was utilized as a co-reactant. Under these conditions, conversion of 1-chloro-1,1-difluoroethane was 80%, with 100% selectivity for vinylidene fluoride. No 1-chloro-1-fluoroethylene was formed.

U.S. Patent 2,894,043 discloses the dehydrofluorination of 1-chloro-1,1-difluoroethane to 1-chloro-1-fluoroethylene at a temperature of 465-535°C, in a reactor packed with nickel metal helices.

## Summary of the Invention

A process for the preparation of mixtures comprising 1-chloro-1-fluoroethylene and vinylidene fluoride is provided. 1-Chloro-1,1-difluoroethane is contacted in the gaseous phase with a $NiF_2$-containing catalyst at a reaction temperature of from about 200°C to about 700°C. Both vinylidene fluoride and 1-chloro-1-fluoroethylene are thus prepared in a single step. They may be easily separated by standard distillation methods owing to their large difference in boiling points. Vinylidene fluoride boils at -86°C; 1-chloro-1-fluoroethylene boils at -24°C.

The process of the invention is particularly useful for preparing mixtures wherein vinylidene fluoride and 1-chloro-1-fluoroethylene comprise in combination at least about 50 mol%, preferably at least about 60 mol%, of the product mixture formed from the disproportionation of 1-chloro-1,1-difluoroethane.

## Detailed Description of the Invention

Both vinylidene fluoride and 1-chloro-1-fluoroethylene are monomers which may be used for the preparation of useful polymers. Vinylidene fluoride is used in the production of polyvinylidene fluoride. 1-Chloro-1-fluoroethylene may be utilized in the production of elastomeric fluoroolefins. See U.S. Patents 2,777,833 and 2,894,043. The present invention provides a convenient process for producing both vinylidene fluoride and 1-chloro-1-fluoroethylene in a single step.

According to the present invention, a $NiF_2$-containing catalyst is advantageously utilized for the gas phase production of vinylidene fluoride and 1-chloro-1-fluoroethylene from 1-chloro-1,1-difluoroethane. By "$NiF_2$containing catalyst" is meant pure $NiF_2$, or $NiF_2$ contained on a support such as $AlF_3$ and the like. Also included in the definition of "$NiF_2$-containing catalyst" is fluorided $NiCl_2$, which may likewise be optionally

2

contained on a suitable support, e.g. $Al_2O_3$. By "fluorided $NiCl_2$" is meant $NiCl_2$, a substantial portion thereof which has been converted to $NiF_2$ by the catalyst activation sequence as hereinafter described. Thus, while Müller, et al., **Chem. Ing. Tech.** 56 (8), 626-628 (1984) disclose unactivated $NiCl_2$ as the catalyst for the dehydrochlorination of 1-chloro-1,1-difluoroethane, the present invention utilizes $NiF_2$, or $NiCl_2$ which has been appropriately "fluorided".

At temperatures below about 400° C the major products obtained are generally 1,1,1-trifluoroethane and 1-chloro-1-fluoroethylene, with vinylidene fluoride present, but as a minor product. At temperatures above about 400° C, vinylidene fluoride is generally formed at the expense of 1,1,1-trifluoroethane, and the principal products are vinylidene fluoride and 1-chloro-1-fluoroethylene. A small percentage of vinylidene chloride is also observed, presumably from the dehydrofluorination of 1,1-dichloro-1-fluoroethane.

Without wishing to be bound by any theory, it is believed that this product distribution results from a disproportionation process. According to the probable disproportionation mechanism, two moles of 1-chloro-1,1-difluoroethane disproportionate to form one mole each of 1,1,1-trifluoroethane and 1,1-dichloro-1-fluoroethane. 1,1,1-Trifluoroethane dehydrofluorinates to form HF and vinylidene fluoride. 1,1-Dichloro-1-fluoroethane dehydrochlorinates to form HCl and 1-chloro-1-fluoroethylene.

The reaction temperature is from about 200° C to about 700° C, preferably from about 400° C to about 600° C, most preferably from about 500° C to about 600° C. Generally, higher temperatures favor the production of vinylidene fluoride at the expense of 1,1,1-trifluoroethane. Thus, temperatures above about 400° C, most preferably above about 600° C, are preferred to maximize vinylidene fluoride production over 1,1,1-trifluoroethane production.

A small amount of oxygen may be optionally combined with the 1-chloro-1,1-difluoroethane feed as a co-reactant. Thus, oxygen may be combined with the 1-chloro-1,1-difluoroethane in an amount of from about 2% to about 21% by volume, based upon the volume of 1-chloro-1,1-difluoroethane passed over the catalyst. Preferably, the amount of oxygen is from about 4% to about 8%. The oxygen may be supplied in the form of substantially pure oxygen or may be supplied in the form of a gas containing oxygen, such as air or a synthetic mixture of molecular oxygen with mostly inert gases. Where a mixture of gases is employed, the volume of gas supplied should be increased proportionately to take into account the oxygen content of the gas.

The contact time of the reactants with the catalyst may vary considerably. Preferably, the contact time is from about 10 seconds to about 100 seconds, most preferably from about 40 seconds to about 70 seconds.

Air activation of the catalyst before introducing the reactant feed is advantageously conducted to boost catalyst performance. The catalyst may be treated in this manner by flowing air over the catalyst at a temperature from about 100° C to about 1000° C, at an air flow rate of from about 1 to about 10 $cm^3/min$. per $cm^3$ of catalyst, for at least about 24 hours. Preferably, the temperature of the air is from about 200° C to about 700° C.

Where the catalyst comprises fluorided $NiCl_2$, the starting catalyst $NiCl_2$ bed, after air activation substantially as above, is thereafter activated with hydrogen fluoride by flowing hydrogen fluoride over the catalyst bed at elevated temperature in an amount of from about 20% to about 60% by volume in excess of the theoretical amount of hydrogen fluoride required to convert the $NiCl_2$ in the bed to $NiF_2$. By way of example, if the theoretical amount of hydrogen fluoride required to completely convert the $NiCl_2$ to $NiF_2$ is 1 mole, then from about 1.2 to about 1.6 moles of hydrogen fluoride are advantageous passed through the $NiCl_2$ bed.

The process of the present invention is illustrated in the following non-limiting examples.


## EXAMPLE 1


Alumina (200 g, Harshaw 3952) was added portionwise at 45° C into a stirred solution of aqueous hydrogen fluoride (500 ml of 50% HF) over the course of 6 hours. $AlF_3 \cdot 3H_2O$ was precipitated when the solution was left standing at room temperature overnight. To this mixture was added 29.94 g $NiCl_2$ portionwise at room temperature. A light green solid was precipitated out from the mixture. The precipitated $NiF_2/AlF_3$ was filtered, air dried and heated gradually at the following temperatures: (1) 100° C for 2 hours, (2) 150° C for 2 hours, and (3) 175° C for 18 hours. The catalyst was then ground and sieved. Of the resulting 60-100 mesh particles, 22.45 g were loaded into a Hastelloy C tubular reactor. The catalyst was activated at 650° C with continuously flowing air fed into the reactor over the catalyst bed at a rate of 20 $cm^3/min$. for 18 hours. The air-activated catalyst was then activated with hydrogen fluoride by flowing 0.08 g/min. of hydrogen fluoride and 20 cm$^3$/min. of nitrogen for 6 hours at 550°C. A total of 29 g of hydrogen fluoride were flowed over the catalyst. A gas feed consisting of 94.3% 1-chloro-1,1-difluoroethane and 5.7% $O_2$ by volume was introduced into the reactor at 450°C. The reaction products were removed at the bottom of the reactor and then passed up to a scrubbing tower, countercurrent to a liquid alkaline stream of 1-5 N aqueous KOH in order to remove hydrogen fluoride. Other aqueous hydroxides such as NaOH or Ca(OH)$_2$ may be substituted for KOH. The organic product was then passed through a drying tower packed with a drying agent (anhydrous calcium sulfate). The conversion of 1-chloro-1,1-difluoroethane was periodically check by passing product automatically to a gas chromatograph equipped with an electronic integrator. The mass balance was evaluated by passing the outlet gas from the gas chromatograph through a wet test meter. Gas chromatograph analysis indicated a 92.7% conversion of 1-chloro-1,1-difluoroethane. The product distribution is set forth in Table 1.

## EXAMPLE 2

The procedure of Example 1 was repeated except that the reaction temperature was increased to 490°C, and the feed consisted of 77.6% 1-chloro-1,1-difluoroethane and 22.4% air, by volume. The results are tabulated in Table 1.

## EXAMPLE 3

The procedure of Example 2 was repeated except that the reaction temperature was increased to 525°C. The results are tabulated in Table 1.

## EXAMPLES 4-6

NiF$_2$•4H$_2$O (32 g) was loaded into the same reactor utilized in Example 1 and heated to 600°C. The catalyst was treated with flowing air at 20 cm$^3$/min. for 18 hours. Thereafter 1-chloro-1,1-difluoroethane (18 cm$^3$/min.) together with air (5.2 cm$^3$/min.) were continuously fed over the catalyst bed at 400°C, 450°C and 575°C. The results appear in Table 1.

## EXAMPLES 7-8

NiCl$_2$•6H$_2$O (32 g, 0.25 mole) was loaded into the same reactor used in Example 1. The catalyst was heated to 200°C using 20 cm$^3$/min. air for 21 hours. The air-activated catalyst was thereafter treated by flowing hydrogen fluoride (0.03 g/min.) up to a total of 11 g, in combination with 20 cm$^3$/min. nitrogen at 400°C. Upon completion of the catalyst activation, a mixture of 92.4% 1-chloro-1,1-difluoroethane and 7.6% oxygen by volume was fed to the reactor at temperatures of 400°C and 450°C. The results are tabulated in Table 1.

## EXAMPLES 9-12

NiCl$_2$•6H$_2$O (30 g) were dissolved in 200 ml of hot water with continuous stirring. Gamma-alumina (100 g) was added to the hot green solution. The mixture was left overnight at room temperature. The mixture was thereafter filtered and air dried. The air dried catalyst (50 g) was loaded onto the same reactor utilized in Example 1. The catalyst was activated at 650°C with air flowing at a rate of 20 cm$^3$/min. for 60 hours. The catalyst was thereafter treated with hydrogen fluoride (0.2 g/min.) for 6 hours, resulting in a total 72 g of hydrogen fluoride being passed over the catalyst bed. A mixture of 91.9% 1-chloro-1,1-difluoroethane and 8.1% oxygen by volume was introduced into the reactor. The results are set forth in Table 1.

TABLE 1

| Catalyst | Example No. | Reactor Feed (vol.%) | | | T˚C | Running Time (hrs.) | Conv. of $CH_3ClF_2$ (mole%) | Product Distribution (mole%) | | | | | |
|---|---|---|---|---|---|---|---|---|---|---|---|---|---|
| | | $CH_3ClF_2$ | Air | $O_2$ | | | | $CH_2=CF_2$ | $CH_3CCl_2F$ | $CH_3CF_3$ | $CH_2=CClF$ | $CH_2=CCl_2$ | Other |
| NiF$_2$/AlF$_3$ | 1 | 94.3 | - | 5.7 | 450 | 6 | 92.7 | 24 | 0.41 | 34.5 | 35.9 | 4.2 | 1 |
| " | 2 | 77.6 | 22.4 | - | 490 | 10 | 96.7 | 32.5 | 0.05 | 24.6 | 38.11 | 3.3 | 1.44 |
| " | 3 | " | " | - | 525 | 6 | 98.7 | 24.4 | 0 | 27.2 | 42.5 | 3.9 | 2 |
| NiF$_2$ | 4 | " | " | - | 400 | 6 | 85.7 | 44.7 | 0 | 28.6 | 23.6 | 2.9 | 0.22 |
| " | 5 | " | " | - | 450 | 66 | 80.5 | 57.5 | 0 | 14.8 | 23.6 | 1.9 | 2.5 |
| " | 6 | " | " | - | 475 | 6 | 89.5 | 47.8 | 0 | 16.8 | 27 | 2.5 | 3.9 |
| Fluorided NiCl$_2$ | 7 | 92.4 | - | 7.6 | 400 | 20 | 51 | 54 | 0.5 | 17.3 | 25.4 | 1.3 | 1.5 |
| " | 8 | " | - | " | 450 | 20 | 70.6 | 55.42 | 0.2 | 14.6 | 27.8 | 0.23 | 1.75 |
| Fluorided NiCl$_2$/Al$_2$O$_3$ | 9 | 91.9 | - | 8.1 | 400 | 17 | 93 | 18.1 | 0.7 | 46 | 26 | 5.7 | 3.4 |
| " | 10 | " | - | " | 450 | 17 | 97.1 | 24.3 | 0.3 | 36.9 | 24.3 | 8 | 6.2 |
| " | 11 | " | - | " | 550 | 13 | 98.2 | 38.9 | 0.5 | 22.8 | 33.5 | 2.4 | 1.9 |
| " | 12 | " | - | " | 600 | 18 | 98.8 | 53.5 | 0.1 | 25.5 | 19.9 | 0.6 | 0.4 |

The present invention may be embodied in other specific forms without departing from the spirit or essential attributes thereof and, accordingly, reference should be made to the appended claims, rather than to the foregoing specification, as indicating the scope of the invention.

## Claims

1. A process for the preparation of mixtures comprising 1-chloro-1-fluoroethylene and vinylidene fluoride comprising contacting 1-chloro-1,1-difluoroethane in the gaseous phase with a $NiF_2$-containing catalyst at a reaction temperature of from about 200°C to about 700°C.

2. A process according to claim wherein the reaction temperature is from about 400°C to about 600°C.

3. A process according to claim 2 wherein the reaction temperature is from about 500°C to about 600°C.

4. A process according to claim wherein the catalyst is $NiF_2$.

5. A process according to claim 4 wherein the catalyst is $NiF_2$ supported on $AlF_3$.

6. A process according to claim wherein the catalyst is fluorided $NiCl_2$.

7. A process according to claim 6 wherein the catalyst is fluorided $NiCl_2$ supported on $Al_2O_3$.

8. A process according to claim 1 wherein the 1-chloro-1,1-difluoroethane is in contact with the catalyst for from about 10 seconds to about 100 seconds.

9. A process according to claim 8 wherein the 1-chloro-1,1-difluoroethane is in contact with the catalyst for from about 40 seconds to about 70 seconds.

10. A process according to claim 1 wherein the 1-chloro-1,1-difluoroethane is combined with from about 2% to about 21% oxygen by volume, based upon the volume of said 1-chloro-1,1-difluoroethane contacted with the catalyst.

11. A process according to claim 10 wherein the 1-chloro-1,1-difluoroethane is combined with from about 4% to about 8% oxygen by volume, based upon the volume of said 1-chloro-1,1-difluoroethane contacted with the catalyst.

12. A process according to claim 1 wherein the catalyst has been treated prior to contacting the 1-chloro-1,1-difluoroethane by flowing air over the catalyst at a temperature of from about 100°C to about 1000°C, at an air flow rate of from about 1 cm³/min. to about 10 cm³/min., per cm³ of catalyst, for at least about 24 hours.

13. A process according to claim 12 wherein the air temperature is from about 200°C to about 700°C.

14. A process according to claim 12 wherein the catalyst comprises fluorided $NiCl_2$, which catalyst is prepared by flowing air over a catalyst bed comprising $NiCl_2$, and thereafter flowing hydrogen fluoride over the catalyst bed in an amount of from about 20% to about 60% by volume in excess of the theoretical amount of hydrogen fluoride required to convert the $NiCl_2$ in the bed to $NiF_2$.

15. A process according to claim 14 wherein the catalyst comprises fluorided $NiCl_2$ on $Al_2O_3$.

16. A process according to claim wherein at least about 50 mol% of the mixture comprises the combination of 1-chloro-1-fluoroethylene and vinylidene fluoride.

17. A process according to claim 16 wherein at least about 60 mol% of the mixture comprises the combination of 1-chloro-1-fluoroethylene and vinylidene fluoride.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-B-1 253 702 (PENNSALT CHEMICALS CORP.) * Column 1, paragraph 2 * --- | 1-3 | C 07 C 21/18<br>C 07 C 17/34<br>B 01 J 27/128 |
| X | US-A-3 444 251 (L.E. GARDNER) * Columns 1-2 * --- | 1-7 | |
| A,D | US-A-2 894 043 (E.J. PRILL) * Column 4 * ----- | 1-3,8 | |

TECHNICAL FIELDS SEARCHED (Int. Cl.5)

C 07 C 17/00

The present search report has been drawn up for all claims

| Place of search | Date of completion of the search | Examiner |
|---|---|---|
| THE HAGUE | 26-09-1990 | VAN GEYT J.J.A. |

EPO FORM 1503 03.82 (P0401)